Europäisches Patentamt

European Patent Office

Office européen des brevets

(11) Veröffentlichungsnummer: **0 231 145**
**A2**

## EUROPÄISCHE PATENTANMELDUNG

(21) Anmeldenummer: 87810003.1

(22) Anmeldetag: 06.01.87

(51) Int. Cl.⁴: **C 12 M 1/08**
**C 12 M 1/36**

(30) Priorität: 17.01.86 CH 183/86

(43) Veröffentlichungstag der Anmeldung:
05.08.87 Patentblatt 87/32

(84) Benannte Vertragsstaaten: **DE FR GB IT**

(71) Anmelder: **Tschudin & Heid AG**
**Haupstrasse 35**
**CH-4437 Waldenburg (CH)**

(72) Erfinder: **Baerfuss, Achilles**
**Sonnenweg 5**
**CH-4104 Oberwil (CH)**

(74) Vertreter: **Eder, Carl E. et al**
**Patentanwaltsbüro EDER AG Münchensteinerstrasse 2**
**CH-4052 Basel (CH)**

(54) **Einrichtung zum Behandeln von Mikroorganismen und Verfahren zum Betrieb der Einrichtung.**

(57) Die Einrichtung weist eine Kammer (5) mit einem Innenraum (11), der in einem Teilbereich seiner Höhe durch eine Trennwand (13) in zwei Teilräume (11a, 11b) unterteilt ist, und mindestens eine Pumpe (21) auf, mit der im ersten Teilraum (11a) vorhandenes flüssiges, Mikroorganismen enthaltendes Substrat (63) durch den zweiten Teilraum (11b) hindurch nach oben gefördert werden kann, so dass es über einen Überström-Rand (27) am oberen Ende der Trennwand (13) wieder in den ersten Teilraum (11a) zurückfällt. Der den Überström-Rand (27) bildende Trennwandteil (23) ist mit einer Stellvorrichtung (29) höhenverstellbar. Bei der Durchführung eines aeroben, mikrobiologischen Prozesses wird die Höhe des Überström-Randes (27) derart eingestellt, dass die Fallhöhe des vom Überström-Rand (27) in den inneren Teilraum (11a) hinunterfallenden Substrats (63) ausreichend gross ist, um zu verhindern, dass im inneren Teilraum (11a) vorhandener Schaum (73) über den Überström-Rand (27) emporsteigt.

Fig.1

EP 0 231 145 A2

Bundesdruckerei Berlin

**Beschreibung**

Einrichtung zum Behandeln von Mikroorganismen und Verfahren zum Betrieb der Einrichtung

Die Erfindung betrifft eine Einrichtung gemäss dem Oberbegriff des Anspruchs 1.

Solche aus den Schweizerpatentschriften 358 195 und 606 436 bekannte, für die Fermentation dienende Einrichtungen weisen eine Reaktor-Kammer auf, in der eine zur Kammerwandung koaxiale Trennwand angeordnet ist, die einen Höhenbereich des Innenraums der Kammer in zwei Teilräume unterteilt. Beim unteren Ende des inneren Teilraumes ist ein als Förderorgan dienendes Schaufelrad einer Pumpe vorhanden, um das im inneren Teilraum vorhandene, flüssige, Mikroorganismen und Nährstoffe für diese enthaltende Substrat durch den äussern, ringspaltförmigen Teilraum hindurch nach oben zu fördern, so dass es über den vom oberen Ende der Trennwand gebildeten Überström-Rand wieder in den inneren Teilraum zurückgelangt. Die Einrichtung weist ferner einen Gaseinlass zum Einleiten von Luft in das flüssige Substrat und einen Gasauslass zum Ableiten des vom Substrat freigesetzten Gasgemischs auf. Beim Umwälzen des Mikroorganismen, gelöste Gase und feine, mehr oder weniger gleichmässig verteilte Gasblasen enthaltenden Substrats entstehen in diesem auch grosse Blasen, die sich in der oberen Zone des im inneren Teilraum vorhandenen Substrats ansammeln und dort Schaum bilden, dessen Niveau bei der aus der Schweizerpatentschrift 606 436 bekannten Einrichtung mit einer Niveau-Sonde ermittelt werden kann.

Beim Betrieb einer solchen Einrichtung wird üblicherweise eine Charge Nährstoff enthaltendes Substrat in die Kammer des Reaktors eingebracht und verarbeitet. Die Menge der eingebrachten Substrat-Charge wird dabei derart bemessen, dass ihr Niveau im inneren Teilraum unter dem Überström-Rand liegt und das diesen beim Betrieb überströmende Substrat in den inneren Teilraum hinunter fällt. Im Verlauf des Kultivierungsvorganges vermehren sich die im Substrat vorhandenen Mikroorganismen. Ferner geben die Mikroorganismen in den meisten Fällen nebst Gas noch mindestens einen festen oder flüssigen Stoff an das Substrat ab, so dass das vom Mikroorganismen, Gase und sonstige Stoffe enthaltenden Substrat eingenommene Volumen zunimmt und sein Niveau ansteigt. Dadurch nimmt die Fallhöhe des vom Überström-Rand in den inneren Teilraum fallenden Substrats ab, wodurch der mikrobiologische Prozess gehemmt wird. Vor allem aber besteht bei abnehmender Fallhöhe die Gefahr, dass der Schaum nach oben aus dem inneren Teilraum heraus gelangt, den ganzen Kammer-Innenraum ausfüllt und durch den Gasauslass aus der Kammer abströmt. Wenn diesem Anwachsen des Schaums nicht entgegengewirkt wird, könnte unter Umständen, etwa bei der Eiweissherstellung, praktisch das ganze Substrat und Produkt in Form von Schaum durch den Gasauslass entweichen. Dies verursacht nicht nur einen Produktverlust, sondern auch die Ansiedlung und Entwicklung von Mikroorganismen der im Substrat vorhandenen Art und von anderen Arten im Gasauslass. Zudem können Mikroorganismen aus dem Gasauslass in die Reaktor-Kammer zurückgelangen und in dieser eine Infektion verursachen.

Zur Vermeidung eines zu hohen Anstiegs des Substrat-Niveaus und des Schaums hat man bisher beim Betrieb von bekannten Einrichtungen der genannten Art im Verlauf des Kultivierungsprozesses ein- oder mehrmals Mikroorganismen enthaltendes Substrat durch einen beim unteren Ende der Kammer vorhandenen, absperrbaren Auslass aus der Kammer abgeleitet und/oder dem Substrat ein der Schaumbildung entgegenwirkendes, die Oberflächenspannung des Substrats reduzierendes Entspannungsmittel beigefügt. Wenn jedoch der Auslass für eine Teilentleerung geöffnet wird, besteht die Gefahr, dass andere Mikroorganismen in den Kammer-Innenraum eindringen und das in diesem verbleibende Substrat infizieren. Um diese Gefahr möglichst gering zu halten, muss der Auslass bei vielen Anwendungen vor jeder Teilentleerung steril gemacht werden. Dies verursacht jedoch erhöhte Umtriebe und ist zudem nur unvollkommen möglich, weil ja die im Kammer-Innenraum vorhandenen Mikroorganismen nicht zerstört werden dürfen. Die Zugabe von Entspannungsmitteln bringt ebenfalls Nachteile mit sich, weil die Verkleinerung der Oberflächenspannung des Substrats den Gasaustausch zwischen Substrat und Mikroorganismen beeinträchtigt, weil die Entspannungsmittel sich an den Oberflächen der Kammerwandung sowie der Trennwand niederschlagen und die beim Aufarbeiten des Produktes verwendeten Filter verstopfen können und meistens mit chemischen Verfahren wieder entfernt werden müssen und weil die Entspannungsmittel sowie deren Entfernung beträchtliche Mehrkosten verursachen können.

Es ist ferner bekannt, Rührwerke aufweisende, mikrobiologische Reaktoren mit Vorrichtungen zur mechanischen Schaumzerstörung und -abscheidung auszurüsten. Diese Vorrichtungen weisen eine Zentrifuge auf, mit der der Schaum zerschlagen und in Flüssigkeit und Gas zerlegt wird. Solche Vorrichtungen sind jedoch teuer und verbrauchen beim Betrieb viel Energie.

Bei der Durchführung anaerober mikrobiologischer Prozesse entsteht zwar normalerweise kein Schaum. Aber das Substrat-Volumen kann auch bei solchen Prozessen so stark zunehmen, dass man vor der Beendigung des Prozesses Substrat aus der Reaktor-Kammer herauslassen muss.

Der Erfindung liegt nun die Aufgabe zugrunde, eine zum Behandeln, insbesondere Kultivieren von Mikroorganismen dienende Einrichtung zu schaffen, die Nachteile der bekannten Einrichtungen behebt und insbesondere ermöglicht, Teilentleerungen während den Behandlungsvorgängen und/oder die Zugabe von Entspannungsmitteln unnötig zu machen oder zumindest weitgehend zu reduzieren. Ferner sollen die Behandlung der Mikroorganismen und der dabei stattfindende mikrobiologische Prozess mit möglichst geringem Energieverbrauch durchgeführt werden können und die Einrichtung kostengünstig herstellbar sein.

Diese Aufgabe wird durch eine Einrichtung der einleitend genannten Art gelöst, wobei die Einrichtung erfindungsgemäss durch den kennzeichnenden Teil des Anspruchs 1 gekennzeichnet ist. Vorteilhafte Ausgestaltungen der Einrichtung gehen aus den vom Anspruch 1 abhängigen Ansprüchen hervor.

Die Erfindung betrifft ferner ein Verfahren nach dem Oberbegriff des Anspruchs 7, des erfindungsgemäss durch den kennzeichnenden Teil dieses Anspruchs gekennzeichnet ist. Günstige Weiterbildungen des Verfahrens ergeben sich aus den von diesem Anspruch abhängigen Ansprüchen.

Die Erfindung wird nun anhand eines in der Zeichnung dargestellten Ausführungsbeispiels erläutert. In der Zeichnung zeigen

die Figur 1 einen schematischen Vertikalschnitt einer Einrichtung zum Kultivieren von Mikroorganismen und

die Figur 2 einen der Figur 1 entsprechenden Vertikalschnitt durch einen Teil der Einrichtung, wobei sich aber der Überström-Rand der Einrichtung höher oben befindet.

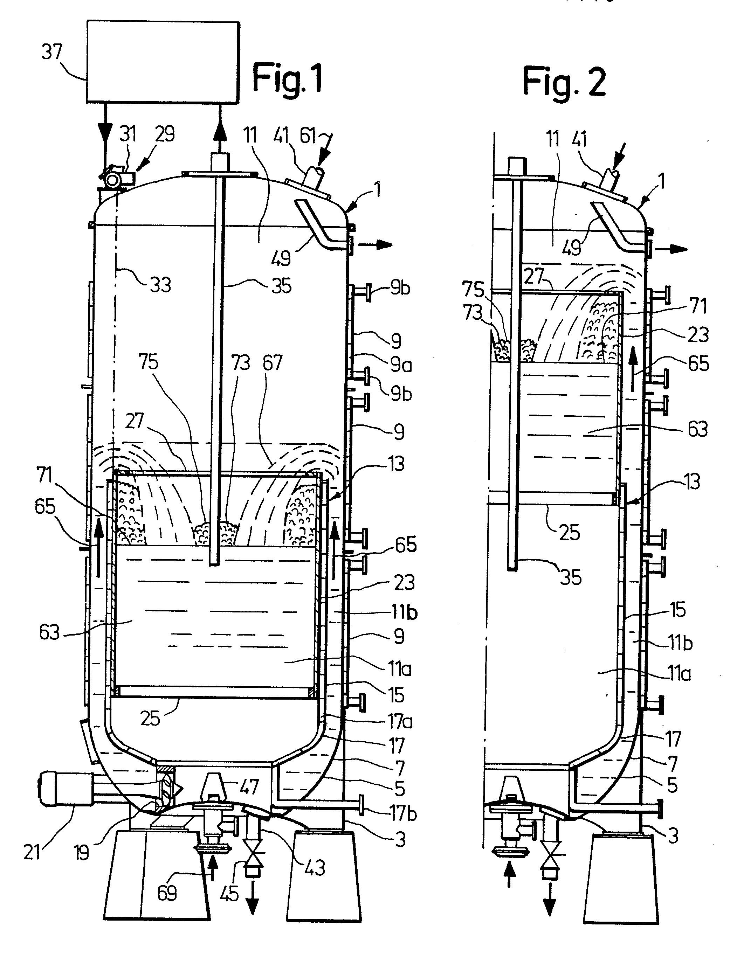

Die in der Figur 1 dargestellte, zum Behandeln, insbesondere Kultivieren und Entwickeln von Mikroorganismen in einem flüssigen Substrat dienende Einrichtung weist einen Reaktor 1 mit einem nur vereinfacht dargestellten Gestell 3 und einer von diesem gehaltenen Kammer 5 auf. Die Wandung 7 der letzteren ist im allgemeinen rotationssymmetrisch zu einer vertikalen Symmetrieachse und hat als Hauptbestandteil einen zylindrischen Wandteil, der an seinem unteren Ende durch einen verrundeten Boden und an seinem oberen Ende durch eine gewölbte Decke abgeschlossen ist, die durch einen lösbar am zylindrischen Wandteil befestigten Deckel gebildet ist. Der zylindrische Wandteil ist mit mindestens einer, beispielsweise mit drei übereinander angeordneten Vorrichtungen 9 zum Kühlen oder eventuell zum wahlweise Kühlen oder Heizen versehen, die je einen beispielsweise wendelförmigen Durchgang 9a für ein Kühl bzw. Heizfluid sowie zwei Anschlüsse 9b zum Ein- bzw. Auslassen dieses Fluids besitzen.

Der untere Höhenbereich des Innenraums 11 der Kammer 5 ist durch eine mehrteilige, zur Kammerwandung 7 koaxiale Trennwand 13 in einem ersten, inneren, einen vollen Querschnitt aufweisenden Teilraum 11a und einen zweiten äusseren, im Querschnitt ringförmigen Teilraum 11b unterteilt. Die Trennwand 13 weist einen Trennwandteil 15 mit einem oben offenen, zylindrischen Hauptabschnitt auf, der an seinem unteren Ende über einen sich verjüngenden Übergangsabschnitt mit einem dünneren, zylindrischen Hals verbunden ist, der starr und dicht am Boden der Kammerwandung 7 befestigt ist. Der zylindrische Hauptabschnitt und der Übergangsabschnitt können im Bedarfsfall mit einer zum Kühlen oder eventuell zum wahlweisen Kühlen oder Heizen dienenden Vorrichtung 17 versehen sein. Diese weist einen beispielsweise wendelförmigen Durchgang 17a für ein Kühl- bzw. Heizfluid und zwei zum Ein- und Auslassen von diesem dienende Anschlüsse 17b auf, wobei die letzteren, von denen nur der eine sichtbar ist, vorzugs weise im Bereich des Bodens der Kammer 5 aus dieser herausgeführt sind. Der Hals des Trennwandteils 15 ist mit mindestens einem Durchgang 19 und beispielsweise mit mehreren, nämlich etwa fünf gleichmässig über seinen Umfang verteilten Durchgängen 19 versehen, die die unteren Enden der beiden Teilräume 11a, 11b fluidmässig verbinden und je ein Förderorgan, nämlich ein Flügelrad einer eine Fördervorrichtung bildenden Pumpe 21 enthalten. Die fünf je mit einem Elektromotor versehenen Pumpen 21 bilden zusammen Fördermittel zum Umwälzen des im Kammer-Innenraum 11 vorhandenen, flüssigen Substrats.

Im zylindrischen Hauptabschnitt des starr an der Kammerwandung 7 befestigten Trennwandteils 15 ist ein Trennwandteil 23 vertikal verschiebbar geführt. Dieser besteht zur Hauptsache aus einem zylindrischen, beidenends offenen Mantel, der an seinem unteren Ende mit einem Verstärkungsring 25 verstärkt ist und an seinem oberen Ende einen radial zur Kammerachse hin nach innen ragenden Kragen aufweist. Dessen obere Begrenzungsfläche bildet einen in einer zur Kammerachse rechtwinkligen, horizontalen Ebene liegenden Überström-Rand 27 für das in der Kammer 5 umgewälzte, flüssige Substrat. Der verstellbare Trennwandteil 23 soll einigermassen dicht am feststehenden Trennwandteil 15 anliegen, so dass beim Umwälzen des Substrates nur verhältnismässig wenig Substrat zwischen den beiden Trennwandteilen 15, 23 hindurchdringt. Es könnten jedoch auch Dichtungsmittel vorgesehen werden, um die beiden Trennwandteile 15, 23 vollständig dicht gegeneinander abzudichten.

Zum Verstellen des vertikal verschiebbaren Trennwandteils 23 ist eine muskelkraftfrei arbeitende Stellvorrichtung 29 vorhanden. Diese weist mehrere, beispielsweise drei gleichmässig um die Mittelachse der Kammer 5 herum verteilte, an der Decke der Kammerwandung 7 angeordnete Stellorgane 31 auf, von denen in der Figur 1 jedoch nur eine dargestellt ist. Jedes Stellorgan 31 hat eine aussen an der Decke der Kammerwandung 7 befestigte Antriebsvorrichtung mit einem drehrichtungssteuerbaren Elektromotor und eventuell noch einem Getriebe. Die horizontale Welle des Motors bzw. die horizontale Abtriebswelle des Getriebes ragt durch eine fluiddichte Durchführung in einen fluidmässig mit dem Kammer-Innenraum 11 verbundenen Raumbereich hinein und ist dort mit einem Wickelelement versehen. Dieses besteht aus einem Kettenrad oder einer Seiltrommel und greift an einem aus einer Kette bzw. Seil bestehenden Zugelement 33 an, dessen eines Ende am oberen Ende des verstellbaren Trennwandteils 23 befestigt ist.

Die Decke der Kammer-Wandung 7 hat in ihrem Zentrum eine mit einem lösbar befestigten Flansch verschlossene Öffnung, an dem eine Niveau-Sonde 35 befestigt ist. Diese hat einen entlang der Symmetrieachse der Kammer 5 senkrecht in den inneren Teilraum 11a hinabragenden stabförmigen, kapazitiven Messwandler, der sich bis in den vom feststehenden Trennwandteil 15 umschlossenen Bereich des Teilraums 11a erstreckt. Die Niveau-Sonde 35 ist elektrisch mit einem Eingang einer elektronischen, vorzugsweise einem Prozessrechner aufweisenden Steuer- und/oder Regelvorrichtung 37 verbunden, die einen elektrisch mit den Stellorganen 31 verbundenen Ausgang hat.

Die Kammer 5 ist bei ihrer Decke mit einem beispielsweise absperrbaren Einlass 41 für das Substrat und bei ihrem Boden mit einem ein Absperrorgan 45 aufweisenden Auslass 43 für das Substrat und das hergestellte Produkt versehen. Ferner mündet ein vorzugsweise absperrbarer Gaseinlass 47 mit einem Gasverteiler in den unteren Bereich des inneren Teilraums 11a. Vom obersten Bereich des Kammer-Innenraums 11 führt ein Gasauslass 49 aus der Kammer heraus.

Zur Durchführung eines mikrobiologischen Prozesses im Reaktor 1 wird eine Charge flüssiges Substrat durch den Einlass 41 in die Kammer 5 eingebracht, wie es durch einen gestrichelten Pfeil 61 angedeutet ist. Die Menge der Substrat-Charge wird so bemessen, dass das in der Kammer 5 vorhandene Substrat 63 30% bis 50% des Kammer-Innenraums 11 füllt. Das Substrat besteht im allgemeinen zur Hauptsache aus Wasser und mindestens einem mit diesem vermischten, flüssigen oder im Wasser gelösten oder eventuell im Wasser suspendierten Nährstoff. Die zur Durchführung des Prozesses benötigten Mikroorganismen können für die erste Charge beispielsweise zusammen mit dem Substrat oder separat ebenfalls durch den Einlass 41 in den Kammer-Innenraum eingebracht werden. Zum Behandeln und insbesondere Kultivieren und Vermehren der Mikroorganismen wird das diese und Nährstoff enthaltende Substrat 63 mit dem aus den fünf Pumpen 21 bestehenden Fördermittel umgewälzt, nämlich unten aus dem ersten, inneren Teilraum 11a abgesaugt und durch den zweiten im Querschnitt ringförmigen, äussern Teilraum 11b in der durch Pfeile 65 angedeuteten Weise nach oben gepumpt, wonach es über den Überström-Rand 27 strömt und wieder in den innern Teilraum 11a hinunterfällt, wobei es einen die Mittelachse der Kammer 5 lückenlos umschliessenden, tromben- und/oder ringförmigen Strahl 67 bildet. Der Reaktor 1 ist vor allem zur Durchführung aerober Prozesse bestimmt, bei denen den Mikroorganismen Sauerstoff zuzuführen ist. Zu diesem Zweck wird beim Umwälzen des Substrats 63 ein durch einen Pfeil 69 angedeutetes, sauerstoffhaltiges Gasgemisch, nämlich Luft, oder eventuell reiner Sauerstoff durch den Gaseinlass 47 hindurch in das Substrat 63 eingeleitet. Die Mikroorganismen nehmen beim Prozess Sauerstoff auf und geben bei den meisten Prozessen ein anderes Gas, beispielsweise Kohlendioxyd und/oder Stickstoff und/oder Stickstoffverbindungen ab. Das nach oben aus dem Substrat in den substrafreien Bereich des Kammer-Innenraums 11 austretende Gas oder Gasgemisch wird durch den Gasauslass 49 aus dem Kammer-Innenraum 11 abgeleitet, wie es durch einen Pfeil bezeichnet ist.

Der Reaktor 1 kann beispielsweise verwendet werden, um aus Kohlenwasserstoff mittels Mikroorganismen Protein herzustellen. Der Kohlenwasserstoff kann dem Substrat in Form von Methanol zugegeben werden, das als Nährstoff für die Mikroorganismen dient. Die Proteinherstellung ist stark exotherm, so dass es in diesem Fall zweckmässig ist, zur Kühlung des Substrats 63, die Kammerwandung 7 im vom Substrat 63 eingenommenen Höhenbereich mit einer oder mehreren der Vorrichtungen 9 und den Trennwandteil 15 mit der Vorrichtung 17 zu kühlen. Bei anderen Prozessen ist es eventuell ausreichend, lediglich die Kammerwandung zu kühlen oder von einer Kühlung abzusehen oder die Vorrichtungen 9, 17 sogar zum Erwärmen zu verwenden.

Die Luft oder der Sauerstoff die bzw. der in das flüssige Substrat 63 eingeleitet werden, wird zum Teil in diesem gelöst und bildet zum Teil feine Blasen deren Durchmesser höchstens etwa 1 mm oder weniger beträgt. Bei den Grenzflächen bei denen das flüssige Substrat an den sich darüber befinden den Innenraumbereich angrenzt, kann sich Schaum bilden, der grosse Blasen mit Durchmessern von mehreren Millimetern oder sogar Zentimetern aufweist. Im Bereich des zweiten, äusseren Teilraumes 11b und des den Überström-Rand 27 überströmenden Substrats entstehender Schaum wird vom überströmenden und den Strahl 67 bildenden Substrat fortlaufend in den ersten, inneren Teilraum 11a abtransportiert und dem dort entstehenden Schaum beigefügt. Auf der oberen Grenzfläche 71 des im inneren Teilraum 11a vorhandenen Teils des Substrats 63 bildet sich daher eine aus Schaum 73 bestehende Schicht, deren obere Grenzfläche mit 75 bezeichnet ist.

Bei der Kultivierung der Mikroorganismen entsteht fortwährend neuer Schaum, so dass die Schaumschicht die Tendenz hat, dicker zu werden und nach oben zu wachsen. Der vom Überström-Rand 27 in den ersten inneren Teilraum 11a herunterströmende oder fallende Substrat-Strahl 67 unterteilt die obere Grenzfläche 71 des flüssigen Substrats 63 und den sich darüber befindenden, Schaum 73 enthaltenden Abschnitt des inneren Teilraums 11a in einen von der Kammer-Mittelachse durchdrungenen Zentralbereich und einen an die Trennwand 13 angrenzenden, ringförmigen Randbereich. Der Strahl 67 kann die Schaumblasen, die er aus den an ihn angrenzenden Raumbereichen mit nach unten reisst und/oder auf die er aufprallt, zerschlagen und zerstören. Bei diesem Vorgang werden Schaumblasen in flüssiges, nach unten zum restlichen Substrat 63 transportiertes Substrat und nach oben entweichendes Gas getrennt. Wenn der Strahl 67 genügend nahe an die Mittelachse der Kammer heranreicht und die Steilheit sowie die Strömungsgeschwindigkeit beim unteren Bereich des Strahls 67 genügend gross sind, kann erreicht werden, dass das Niveau des Schaumes 73, d.h. die Höhe der Grenzfläche 75, im genannten Zentralbereich unterhalb des Überström-Randes 27 bleibt. Im zwischen dem Strahl 67 und der Trennwand 13 vorhandenen Randbereich kann der Schaum höher ansteigen und unter Umständen bis zur unteren Begrenzung des Überström-Randes 27 gelangen, jedoch nicht durch den Strahl 67 hindurch nach oben dringen. Wenn also das den Überström-Rand 27 überströmende und von diesem in den inneren Teilraum 11a hinunterfallende Substrat eine genügende Fallhöhe hat, verhindert es im ganzen Querschnitt des ersten, inneren Teilraums 11a, dass Schaum aus diesem herausdringt.

Die Niveau-Sonde 35 ermittelt laufend bei der Kammer-Mittelachse das Niveau des Schaums 73, d.h. die Höhe der Grenzfläche 75, die die Schicht des aus Substrat und Gasblasen bestehenden Schaums 73 oben begrenzt. Falls kein Schaum vorhanden sein sollte, ermittelt die Sonde 35 das Niveau des flüssigen Substrats 63, d.h. die Höhe der Grenzfläche 71. Im übrigen sind das schaumfreie, aber ja auch feine Gasblasen enthaltende Substrat 63 und der Schaum 73 in Wirklichkeit nicht unbedingt so scharf gegeneinander abgegrenzt, wie es in der Zeichnung dargestellt wurde, und können in einem Über gangsbereich ineinander übergehen. Die Sonde 35 übermittelt das erfasste Niveau in Form elektrischer, analoger oder digitaler Signale an die Steuer- und/oder Regelvorrichtung 37.

Wenn die Mikroorganismen sich im Verlauf ihrer Kultivierung vermehren und eventuell nebst Gasen auch flüssige oder feste Stoffe an das Substrat abgeben, vergrössert sich im allgemeinen das von diesem eingenommene Volumen. Diese Volumenvergrösserung liegt typischerweise im Bereich von 10% bis 35%, kann aber je nach Art des Prozesses auch ausserhalb dieses Bereiches liegende Werte annehmen und bis 50% oder sogar noch mehr betragen. Wenn der im Substrat vorhandene Anteil von Mikroorganismen und/oder der von diesen erzeugten Substanzen ansteigt, nimmt meistens auch noch die Viskosität des Substrats zu, was eventuell die Schaumbildung verstärken kann.

Die Steuer- und/oder Regelvorrichtung 37 steuert und/oder regelt die Stellorgane 31 der Stellvorrichtung 29 in Abhängigkeit des mit der Niveau-Sonde 35 ermittelten Niveaus derart, dass der Überström-Rand 27 immer um eine Höhendifferenz über der Grenzfläche 75 bzw. beim allfälligen Fehlen von Schaum über der Grenzfläche 71 liegt und dass diese Höhendifferenz, mindestens gleich einem zur Schaumzerstörung notwendigen Mindestwert ist, so dass der im ersten Teilraum 11a vorhandene Schaum 73 und natürlich auch das im ersten Teilraum 11a vorhandene Substrat 63 während der ganzen Zeitdauer, in der das Substrat in der Kammer 5 umgewälzt und/oder Gas in dieses eingeleitet wird, nie über den ersten Teilraum 11a hinaussteigen. Wenn also das Volumen des suspendierte Mikroorganismen und eventuell noch von diesen produzierte Stoffe enthaltenden Substrats zunimmt, hebt die Stellvorrichtung 29 den Trennwandteil 23 nach oben, so dass dieser beispielsweise in die in der Figur 2 dargestellte Höhe gelangen kann. Da die Pumpen 21 das Substrat ungefähr um die zwischen der oberen Grenzfläche 71 des Substrats und dem Überström-Rand 27 vorhandene Höhendifferenz nach oben fördern müssen, soll die Höhendifferenz zwischen dem Überström-Rand 23 und der Grenzfläche 75 vorteilhafterweise auch nicht viel grösser als der erwähnte Mindestwert, sondern möglichst ungefähr gleich diesem sein. Wenn diese Bedingung erfüllt ist, kann einerseits ein Ansteigen der Schaummenge und andererseits ein unnötiger Energieverbrauch der Pumpen 21 vermieden werden.

Der vorzugsweise in der Vorrichtung 3 vorhandene Prozessrechner kann beispielsweise derart ausgebildet und programmiert sein, dass die Höhe des Überström-Randes 27 derart eingestellt wird, dass der letztere während der ganzen Zeitdauer, während der das Substrat umgewälzt und belüftet wird, um eine innerhalb eines gewissen Toleranzhöhenbereichs zeitlich konstante Höhendifferenz über dem Niveau der oberen Schaum-Grenzfläche 75 bzw. im Fall, dass kein oder noch kein Schaum vorhanden ist, über dem Niveau der oberen Substrat-Grenzfläche 71 liegt. Der Überström-Rand 27 kann jedesmal, wenn die genannte Höhendifferenz den unteren, vorgegebenen Grenzwert erreicht, um den Toleranzhöhenbereich auf einen oberen, vorgegebenen Grenzwert angehoben werden. Wenn also das Volumen des Substrats im Verlauf des mikrobiologischen Prozesses zunimmt, wird der Überström-Rand 27 schrittweise angehoben. Falls das Substrat-Volumen abnehmen sollte, was jedoch normalerweise nicht der Fall ist, solange keine Entleerung vorgenommen wird, kann die Steuer- und/oder Regelvorrichtung 37 selbstverständlich auch ein Absenken des Überström-Randes 27 bewirken.

Unter Umständen kann die zwischen dem Substrat- bzw. Schaum-Niveau und dem Überström-Rand 27 vorhandene Höhendifferenz auch nur während eines Teils des Umwälzvorgangs innerhalb des genannten Soll-Toleranzhöhenbereichs gehalten werden. Das Substrat- und Schaum-Niveau könnten beispielweise während der Anfangsphase der Verarbeitung einer Substrat-Charge, wenn das Substrat-Volumen noch relativ klein ist, so tief unter der tiefsten, einstellbaren Höhe des Überström-Randes liegen, dass die genannte Höhendifferenz grösser als der vorgesehene, obere Grenzwert ist.

Es könnte jedoch auch vorgesehen werden, dass die Höhendifferenz zwischen der dem Überström-Rand 27 und der Grenzfläche 75 bzw. 71 und dementsprechend der untere sowie obere Höhendifferenz-Grenzwert im Verlauf des Prozesses verändert werden, um die Höhendifferenz an die ändernde Viskosität und/oder andere ändernde Eigenschaften des Substrats anzupassen, die die Schaumbildung und/oder dessen Zerstörung und Abbau durch das überströmende Substrat beeinflussen. Die einzustellende Höhendifferenz könnte beispielsweise in Abhängigkeit von der Höhe der Grenzfläche 73 vorgegeben werden. Dabei besteht auch die Möglichkeit, eine feste oder eine in Abhängigkeit von der Höhe der Grenzfläche 75 abhängige Höhendifferenz für verschiedene Produkte verschieden festzulegen. Die einzustellende Höhendifferenz zwischen dem Überström-Rand 27 und der oberen Grenzfläche 75 kann beispielsweise durch einige Versuche ermittelt oder berechnet werden. Im übrigen kann der Prozessrechner eventuell auch "lernfähig" programmiert sein, so dass er beispielsweise beim erstmaligen Erzeugen eines bestimmten Produkts die optimale Höhendifferenz und deren allenfalls vorzunehmende Änderung selbst ermittelt und dann speichert.

Die Einrichtung kann ferner noch elektrisch mit der Steuer- und/oder Regelvorrichtung 37 verbundene Sensoren aufweisen, um die Stellung des vertikal verstellbaren Trennwandteils 23 zu erfassen und zu gewährleisten, dass die drei Stellorgane 31 den Trennwandteil 23 gleichmässig heben oder senken und der letztere nicht "verkantet" wird.

Wenn die eingefüllte Charge des Substrats verarbeitet und zumindest teilweise durch die Mikroorganismen umgesetzt wurde, kann man einen Teil des Substrats mitsamt den darin vorhandenen Mikroorganismen und von diesen erzeugten Stoffen durch den Auslass 43 aus dem Reaktor 1 abströmen lassen und die gewünschten Produkte beispielsweise durch Filtrieren abtrennen. Danach kann man wieder eine Charge neues Substrat in die Kammer 5 einbringen. Wenn nacheinander mehrere Substrat-Chargen zur Bildung des gleichen Produkts verarbeitet werden sollen, belässt man beim Chargen-Wechsel vorteilhafterweise jeweils einen Teil der alten Charge in der Reaktor-Kammer 5, um das neu in die Kammer 5 eingebrachte Substrat mit den Mikroorganismen der alten Charge zu impfen. Beim Chargen-Wechsel wird der Überström-Rand selbstverständlich wieder soweit abgesenkt, dass sich für den Substrat-Strahl 67 die vorgesehene Fallhöhe ergibt.

Die Kammer 5 kann beispielsweise einen Durchmesser in der Grösse von 2 bis 5 Metern und eine Höhe im Bereich von 5 bis 10 Metern aufweisen. Der erwähnte Toleranzhöhenbereich beim Einstellen der Höhe des Überström-Randes kann dann etwa im Bereich von 5 bis 10 Zentimetern liegen.

Die Abmessungen der Kammer können selbstverständlich in weiten Grenzen variiert werden, wobei dann auch die Anzahlen und Ausbildungen der Pumpen und Stellorgane angepasst werden können. Bei einem kleineren, für Laborversuche vorgesehenen Reaktor könnte man beispielsweise nur eine einzige Pumpe vorsehen und deren Flügelrad koaxial zur Achse der Reaktor-Kammer im durch den Hals der Trennwand gebildeten, untersten, engsten Abschnitt des inneren Teilraums anordnen. Der innere Teilraum würde in diesem Fall unter dem unteren Rand der Trennwand und um die Kammer-Achse herum durch einen ringspaltförmigen Durchgang mit dem äusseren Teilraum verbunden, ähnlich wie aus der einleitend genannten Schweizerpatentschrift 606 436 bzw. der dieser entsprechenden US-Patentschrift 4 126 517 bekannt ist. Ferner könnte man anstelle der je einen Ketten- oder Seilzug aufweisenden Stellorgane 31 eine Stellvorrichtung mit mindestens einem hydraulischen oder eventuell pneumatischen, einen Zylinder und Kolben aufweisenden Stellorgan und einer elektrisch angetriebenen Druckfluidquelle vorsehen, was insbesondere bei verhältnismässig kleinen Reaktoren vorteilhaft wäre.

Des weiteren könnte der zylindrische Abschnitt des höhenverstellbaren Trennwandteils einen grösseren Durchmesser als der zylindrische Hauptabschnitt des unverschiebbar befestigten Trennwandteils haben und also auf der Aussenseite des letzteren verschiebbar geführt sein.

Insbesondere bei verhältnismässig kleinen Reaktoren könnte ferner der beim in der Figur 1 dargestellten Reaktor am oberen Ende des höhenverstellbaren Trennwandteils vorhandene, radial nach innen ragende Kragen wegfallen, sodass der Überström-Rand dann durch den oberen Rand des zylindrischen Mantels des höhenverstellbaren Trennwandteils gebildet würde.

Die Einrichtung kann zusätzlich zur erwähnten Proteinherstellung auch für viele andere aerobe, mikrobiologische Prozesse verwendet werden. Beispielsweise könnten auch Hefe, Vitamin C oder Enzyme durch mikrobiologische Umsetzungsprozesse hergestellt werden. Dabei können je nach der Art des Prozesses zusätzlich zur Kultivierung von Mikroorganismen auch noch andere Prozesse, etwa chemische Reaktionen, an denen ein von den Mikroorganismen erzeugtes Produkt beteiligt ist, im Reaktor 1 stattfinden.

Eventuell kann die Einrichtung sogar für anaerobe mikrobiologische Prozesse verwendet werden, wobei dann der Gaseinlass und der Gasauslass dicht abgeschlossen würden. Falls eine Einrichtung ausschliesslich für anaerobe Prozesse vorgesehen sein sollte, könnten der Gaseinlass und -auslass überhaupt wegfallen. Bei einem anaeroben Prozess entsteht zwar üblicherweise kein Schaum. Die Höhenverstellbarkeit des Überström-Randes würde in diesem Fall jedoch immer noch ermöglichen, die Höhe des Überström-Randes an das im Verlauf des Prozesses zunehmende Substrat-Volumen anzupassen.

**Patentansprüche**

1. Einrichtung zum Behandeln, insbesondere Kultivieren, von Mikroorganismen in einem flüssigen Substrat, insbesondere für die Durchführung eines aeroben, mikrobiologischen Prozesses, mit einer Kammer (5), einer in dieser angeordneten, deren Innenraum (11) in zwei Teilräume (11a, 11b) unterteilenden, am oberen Ende einen Überström-Rand (27) bildenden Trennwand (13) und Fördermitteln (21), um das Substrat aus dem einen, ersten Teilraum (11a) durch den anderen, zweiten Teilraum (11b) hindurch nach oben zu fördern, so dass es über den Überström-Rand (27) in den ersten Teilraum (11a) zurückströmt, dadurch gekennzeichnet, dass der Überström-Rand (27) höhenverstellbar ist.

2. Einrichtung nach Anspruch 1, gekennzeichnet durch eine Stellvorrichtung (29) zum vertikalen Verstellen des Überström-Randes (27), wobei die Stellvorrichtung (29) derart ausgebildet ist, dass sie den Überström-Rand (27) auch während der Umwälzung des Substrats verstellen kann, und wobei die Stellvorrichtung (29) vorzugsweise mindestens ein muskelkraftfrei, beispielsweise elektrisch und/oder hydraulisch und/oder eventuell pneumatisch arbeitendes Stellorgan (31) aufweist.

3. Einrichtung nach Anspruch 2, mit mindestens einer Niveau-Sonde (35), um das Niveau des im ersten Teilraum (11a) oben auf dem in diesem enthaltenen Substrat (63) vorhandenen Schaums (73) oder, im Fall der Abwesenheit von Schaum, das Niveau des Substrats (63) zu ermitteln, gekennzeichnet durch eine elektronische, beispielsweise einen Prozessrechner aufweisende Steuer- und/oder Regelvorrichtung (37), die mit der Niveau-Sonde (35) und der Stellvorrichtung (29) verbunden ist, um die Höhe des Überström-Randes (27) in Abhängigkeit vom mit der Niveau-Sonde (35) ermittelten Schaum- bzw. Substrat-Niveau einzustellen.

4. Einrichtung nach einem der Ansprüche 1 bis 3, dadurch gekennzeichnet, dass die Trennwand (13) einen starr mit der die Kammer (5) aussen begrenzenden Wandung (7) verbundenen Trennwandteil (15) und einen vertikal verschiebbaren, den Überström-Rand (27) bildenden Trennwandteil (23) aufweist.

5. Einrichtung nach Anspruch 4, dadurch gekennzeichnet, dass der starr mit der Kammerwandung (7) verbundene Trennwandteil (15) einen zylindrischen Abschnitt aufweist, der einen zylindrischen Abschnitt des verstellbaren Trennwandteils (23) führt.

6. Einrichtung nach einem der Ansprüche 1 bis 5 wobei die Trennwand (13) die vertikale Mittelachse der Kammer (5) und den ersten Teilraum (11a) umschliesst und sich der zweite Teilraum (11b) zwischen der die Kammer (5) aussen begrenzenden Wandung (7) und der Trennwand (13) befindet, dadurch gekennzeichnet, dass der Überström-Rand (27) zur Mittelachse der Kammer (5) hin über den unten an ihn anschliessenden, vertikalen Abschnitt der Trennwand (13) vorsteht.

7. Verfahren zum Betrieb der Einrichtung nach einem der Ansprüche 1 bis 6, bei dem ein Mikroorganismen enthaltendes Substrat (63) aus dem ersten Teilraum (11a) durch den zweiten Teilraum (11b) hindurch nach oben gefördert wird, so dass es über den Überström-Rand (27) in den ersten Teilraum (11a) zurückströmt, dadurch gekennzeichnet, dass die Höhe des Überström-Randes (27) derart eingestellt wird, dass im ersten Teilraum (11a) vorhandener Schaum (73) nicht nach oben aus dem ersten Teilraum (11a) heraus dringt.

8. Verfahren nach Anspruch 7, wobei beispielsweise für die Durchführung eines aeroben, mikrobiologischen Prozesses Sauerstoff oder ein sauerstoffhaltiges Gasgemisch in das in der Kammer (5) umgewälzte Substrat (63) eingeleitet sowie Gas oder ein Gasgemisch aus dem sich über dem Substrat befindenden Bereich des Kammer-Innenraums (11) abgeleitet wird und wobei mit einer Niveau-Sonde (35) das Niveau des im ersten Teilraum (11a) vorhandenen Substrat-Schaums (73) oder, falls kein solcher vorhanden ist, des Substrats (63) ermittelt wird, dadurch gekennzeichnet, dass der Überström-Rand (27) mindestens während eines Teils und beispielsweise während der ganzen Substrat-Umwälzdauer auf eine über dem mit der Niveau-Sonde (35) ermittelten Niveau liegende Höhe eingestellt wird.

9. Verfahren nach Anspruch 8, dadurch gekennzeichnet, dass die zwischen dem Überström-Rand (27) und der mit der Niveau-Sonde (35) ermittelten Höhe vorhandene Höhendifferenz mindestens während eines Teils und beispielsweise während der ganzen Substrat-Umwälzdauer auf einem Wert gehalten wird, der mindestens gleich einem unteren und höchstens gleich einem oberen Grenzwert ist, wobei die beiden Grenzwerte beispielsweise während der ganzen Umwälzdauer konstant sind.

0231145

Fig.1          Fig. 2